# EUROPEAN PATENT SPECIFICATION

(11) **EP 4 341 336 B1**
(45) Date of publication and mention of the grant of the patent: **01.01.2025**
(21) Application number: 22725932.2
(22) Date of filing: 16.05.2022
(51) Int. Cl.: C08J 11/24, C07C 67/00, C07C 69/82

(54) **PROCESS FOR DEPOLYMERIZING POLYETHYLENE TEREPHTHALATE BY GLYCOLYSIS**
VERFAHREN ZUR DEPOLYMERISATION VON POLYETHYLENTEREPHTHALAT DURCH GLYKOLYSE
PROCÉDÉ DE DÉPOLYMÉRISATION DE TÉRÉPHTALATE DE POLYÉTHYLÈNE PAR GLYCOLYSE

(30) Priority: 17.05.2021 IT 202100012617
(43) Date of publication of application: 27.03.2024
(73) Proprietor: Aquafil S.p.A., 38062 Arco (IT)
(72) Inventor: CECCHETTO, Michele, 38062 Arco (TN) (IT); BERTOLLA, Maddalena, 38062 Arco (TN) (IT); DAL MORO, Anacleto, 38062 Arco (TN) (IT); MODESTI, Michele, 35131 Padova (PD) (IT); GUERRA, Stefano, 38062 Arco (TN) (IT)
(74) Representative: Bottero, Carlo
(86) International application number: PCT/IB2022/054519
(87) International publication number: WO 2022/243832

(56) References cited:
- WO-A1-2017/087752
- CN-A- 112 646 135
- SU-A1- 146 736
- US-A- 4 163 860
- US-A1- 2012 223 270
- S. R. SHUKLA ET AL: "Glycolysis of polyethylene terephthalate waste fibers", JOURNAL OF APPLIED POLYMER SCIENCE, vol. 97, no. 2, 15 July 2005 (2005-07-15), pages 513 - 517, XP055081732, ISSN: 0021-8995, DOI: 10.1002/app.21769

## Description

The present invention relates to a process for depolymerizing polyethylene terephthalate by glycolysis wherein the catalyst in prepared in situ.

In particular, the process uses catalysts that have a reduced environmental impact and can be prepared from readily available raw materials.

Polyethylene terephthalate (PET) is a semicrystalline thermoplastic polyester with high strength and transparency. PET is obtained by esterification of terephthalic acid (TPA) with ethylene glycol (EG) or transesterification of dimethylterephthalate (DMT) with EG. Thanks to its physical and chemical properties, PET has numerous applications. In particular, PET is used for the production of textile fibers and carpets *(fiber grade PET*), food containers (e.g. beverage bottles - *bottle grade PET*) and films for food packaging *(film grade PET*)*.*

At present, worldwide PET production amounts to more than 80 million tonnes per year. In view of the high consumption and non-biodegradability of PET, there is a strong technical need to recycle this material in order to reduce the environmental impact of end-of-life products and the consumption of raw materials of fossil origin for the manufacture of new products.

A first way of recycling PET waste is through so-called "mechanical recycling". Mechanical recycling is based on the mechanical separation of the polymer from contaminants and its subsequent regranulation. In particular, mechanical recycling includes sorting and separation of PET waste, removal of contaminants, grinding, heat treatment and extrusion into granules. Although widely used, this recycling technique has the limitation that it can only be used to recover PET from bottles, where the content of contaminants is relatively low (less than about 10% w/w). In addition, the presence of dyes and other additives in PET waste and the need to regranulate it to allow it to be reintroduced into industrial production cycles results in a recycled polymer of lower quality than the polymer obtained from virgin raw materials. Mechanical recycling is also associated with the risk of accumulation of the above-mentioned contaminants in the polymer, resulting in a degradation of the quality of the polymer obtained each time it is recycled.

A second way of recycling PET waste is so-called "chemical recycling". Chemical recycling is based on the chemical transformation of the polymer chains of PET into its starting monomers or oligomers by solvolysis (depolymerization). PET depolymerization products can be easily purified from contaminants before being used in new polymerization processes, thus overcoming the drawbacks associated with mechanical recycling processes. As chemical recycling allows the original raw materials to be obtained from the processing of PET waste, this technique has the added advantage of meeting the criteria underlying the concepts of circular economy and sustainable development.

The PET depolymerization reaction can be carried out in several ways:
- via hydrolysis (acid, alkaline or neutral) producing the monomers EG and TPA;
- via methanolysis, producing DMT, which is then transformed into PET by transesterification with EG;
- via glycolysis, e.g. by breaking polymer chains by EG, producing bis-2-hydroxyethyl terephthalate (BHET).

Today, depolymerization by means of glycolysis with EG appears to be the most advantageous recycling technique with the greatest potential for development, as it leads to a product, the compound BHET, which can be used directly as a monomer in the synthesis of PET or other widely used polymers (e.g. unsaturated polyester resins for the production of polyurethanes).

Depolymerization of PET by glycolysis with EG is generally carried out at a temperature in the range 180°C - 250°C and atmospheric pressure, typically in the presence of a catalyst. During depolymerization, the polymer chains are transformed by means of solvolytic cleavage of the polymer chains leading to a theoretically complete depolymerization down to the monomer (BHET) or a partial depolymerization leading to the monomer together with the oligomers.

A schematic representation of the reactions involved in the depolymerization process of PET with EG is given in Diagram 1 below:

### Diagram 1

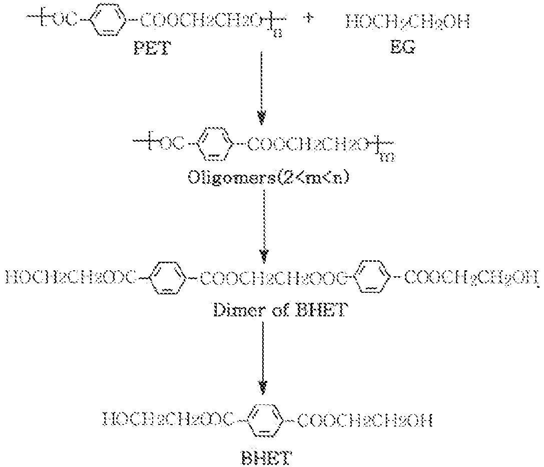

BHET can be used as a monomer to produce PET of various grades *(bottle grade, fiber grade and film grade*) with characteristics and properties fully equivalent to those of PET obtained from virgin raw materials. The polymerization reaction leading to the formation of PET from BHET is shown in Diagram 2:

### Diagram 2

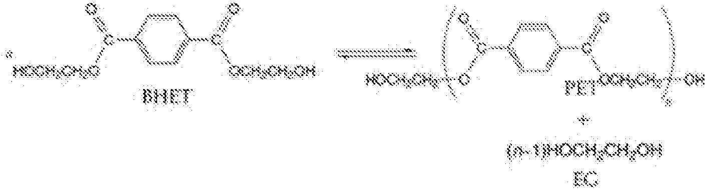

The polymerization of BHET releases a mole of EG, which can be recycled, e.g. in a depolymerization process.

The depolymerization reaction can be carried out in the presence or absence of a catalyst. However, non-catalytic depolymerization is an economically unviable process in view of the low conversion yields of PET to BHET and the significantly longer reaction times compared to catalytic glycolysis.

The most commonly used catalysts for PET depolymerization are mainly based on metal salts, in particular salts of Zn, Ti, Pb, Mn and Na. The salts of these metals are typically acetate, carbonate, bicarbonate and chloride salts. In industrial processes, zinc acetate is by far the most widely used catalyst due to its high efficiency in terms of rate and yield of the depolymerization reaction.

At the end of depolymerization, the metals introduced with the catalyst must be removed from the monomer before it is used for the production of further polymers, both to prevent interference with the quality of the final product and to avoid the accumulation thereof in the polymer during subsequent recycling cycles.

Catalytic depolymerization processes in which metal salts are used as catalysts are described for example in EP 0723951 A1 and WO 2017/087752A1.

EP 0723951 A1 describes a process for preparing high purity BHET by depolymerizing PET from recycled post-consumer waste or post-industrial waste. Preferably, depolymerization is catalyzed by catalysts based on Zn, La and Ce acetates or titanium butoxide (Ti(OBu)₄). The only example of process implementation uses zinc acetate as a catalyst in an amount of approximately 2.2 mmol/kg (PET + EG). EP 0723951 A1 also mentions calcium acetate and other compounds among the catalysts tested, indicating that calcium acetate is poorly effective compared to zinc acetate.

WO 2017/087752 A1 describes a method for chemically recycling PET in which the depolymerization reaction is assisted by microwaves. The reaction mixture comprises a catalytic system comprising a catalyst and a microwave absorber. The catalyst is preferably a zinc salt, in particular zinc acetate. Alternative catalysts include magnesium acetate.

SU 146 736 A1 discloses a method for producing bis(2-hydroxyethyl) terephthalate (BHET) from polyethylene terephthalate by treating PET flakes with ethylene glycol in the presence of magnesium acetate at a temperature of 200°C.

US 4 163 860 A discloses a process, which includes a step of glycolysis of polyester scraps containing 97 % of poly(ethylene terephthalate) carried out using 1 part of ethylene glycol per part of polyester in the presence magnesium acetate at the boiling point of the glycol to obtain a concentrated solution of bis(ethylene glycol) terephthalate.

JP S53 71035 A discloses a method of depolymerising a material comprising polyethylene terephthalate (PET) by glycolysis with ethylene glycol, which comprises reacting the material comprising PET with EG in the presence of at least calcium acetate so that BHET and/or oligomers thereof are obtained.

The article by Shukla et al., entitled "Glycolysis of polyethylene terephthalate waste fibers", JOURNAL OF APPLIED POLYMER SCIENCE, vol. 97, no. 2, 15th July 2005, pages 513-517, describes the glycolysis of polyethylene terephthalate waste fibers carried out using excess ethylene glycol in the presence of glacial acetic acid.

CN 112 646 135 A discloses a method for continuously preparing spinnable colorless recycled polyester from colored waste polyester textiles, wherein colored waste polyester textiles are crushed and melted in the presence of an alcoholysis agent and a catalytic system which can be a metal acetate.

US2014/031441 A1 discloses a process for preparing a modified polybutylene terephthalate from recycled polyethylene terephthalate, the process comprising: in-situ catalyst preparation, depolymerization of recycled PET with ethylene glycol to form bis-hydroxy ethyl terephthalate, ester interchange, and polymerization, wherein the catalyst is the reaction product of tetra(C1-C8 alkyl) titanate and a complexing agent selected from the group consisting of phosphorus-containing compounds, nitrogen-containing compounds, boron-containing compounds, and combinations thereof.

US 2012/223270 A1 discloses a method of preparing monomeric dihydroxy terephthalate diesters by organocatalyzed depolymerization of terephthalate polyesters by employing an amidine salt organo catalyst, which can be generated in-situ, and a glycol comprising 2 to 5 carbons.

The metal catalysts for PET depolymerization currently used in the state of the art have several drawbacks. First of all, the most effective catalysts are based on transition metals and heavy metals (e.g. Zn, Ti, Pb), i.e. elements which have a high environmental impact and whose removal from the BHET monomer at the end of depolymerization is rather difficult.

Secondly, since catalysts are used in relatively large quantities (the catalyst/PET ratio in the reaction mixture can be as high as 4-6 % w/w, depending on the type of catalyst and the reaction conditions), it is crucial in an industrial plant to ensure that large quantities of catalyst are constantly available to guarantee the continuity of the depolymerization process. This implies that the plant is equipped with appropriate storage space for the catalyst, which very often also has to be stored under controlled humidity and temperature conditions to prevent degradation (the acetate salts of alkali and alkaline earth metals, for example, are hygroscopic).

Thirdly, since catalysts are synthetic compounds, the cost of procuring them is relatively high and significantly affects the final cost of the resulting BHET.

In view of the aforementioned drawbacks, it is clear that the state of the art clearly calls for catalytic depolymerization processes using alternative catalysts with respect to those of the known art. In particular, the catalysts must have a low environmental impact and be easily and cheaply procurable.

A further drawback of PET recycling processes is the limited type of waste that can be depolymerized. At present, PET waste recovered by depolymerization is almost exclusively that from the separate collection of plastic containers, particularly bottles. Other types of waste, in particular PET fiber from spinning processes or post-consumer carpet recovery, remain largely unused and destined for landfill due to the high levels of contaminants they contain.

Carpets containing PET, for example, are made up of a multilayer structure comprising different types of materials, polymeric and non-polymeric, in which various inorganic materials used as additives are present. In particular, in carpets, polyester is used to make the externally visible fibrous part, the underlying support fabric or mesh to which the fibrous part is attached (so-called primary backing) or the backing of the carpet in contact with the support surface (so-called secondary backing). Intermediate layers of adhesive or reinforcing materials such as rubber latex and filler materials (e.g. calcium and magnesium salts, aluminium compounds) can be placed between these layers. At the end of their life cycle, carpets containing PET generally undergo shredding and separation of the contaminant fractions, resulting in a fibrous material with a cotton-like appearance (so-called *fluff*). Fluff contains PET together with a relatively high amount of contaminants (up to about 20% by weight), the most abundant of which are: calcium and/or magnesium carbonate, dolomite minerals, aluminosilicates and aluminium tri-hydroxide).

The particular structure and chemical composition of carpets, together with the contaminants absorbed during use (e.g. glues), make the recovery of the PET contained therein rather complex and not economically viable. However, PET waste from carpets remains a highly interesting source of material for recycling due to the high volumes available. It is therefore desirable to develop depolymerization processes that can also make effective use of this type of materials.

In view of the aforementioned state of the art, the Applicant has therefore faced the problem of providing a process for depolymerizing a material comprising PET that overcomes the aforementioned drawbacks of the known art. In particular, the Applicant has faced the problem of providing a depolymerization process that uses catalysts having a reduced environmental impact and efficacy comparable or even superior to that of known catalysts. In addition, catalysts must be readily available or be able to be easily prepared from raw materials with these characteristics.

The Applicant also addressed the issue of providing a depolymerization process to which material comprising PET from various types of waste and process scrap can be supplied, including material comprising PET from waste containing significant amounts of contaminants, such as waste from post-consumer carpet recovery.

It has now been found that the above and other purposes, which will be further illustrated in the following description, can be achieved by a process for depolymerizing a material comprising PET in which a calcium, magnesium or aluminium acetate is used as a catalyst. It has been found that these salts have an efficiency comparable to, and sometimes even higher than, the most efficient catalysts known in the state of the art, such as zinc acetate, while having a reduced environmental impact since the metal component does not consist of heavy or transition metals, but of alkaline earth metals (Ca and Mg) and amphoteric metals (Al). Moreover, these salts, which are readily available and relatively cheap, can be easily removed from the BHET obtained at the end of depolymerization, even by simple washing with water.

In some cases (e.g. calcium acetate), the catalysts according to the present invention exhibit higher reaction rates than known catalysts, even at reduced weight concentrations, allowing depolymerization to be completed in a shorter time with respect to known processes. This offers the advantage of being able to use lower-volume reactors for the same production capacity than those required in state-of-the-art processes.

The Applicant also surprisingly found that the depolymerization catalyst can also be formed *in situ* in the reaction mixture from acetic acid and compounds containing the metal counter-ion, such as oxides, hydroxides, carbonates or bicarbonates of the aforementioned metals. By appropriately dosing the acetic acid and the metal-containing compound in the reaction mixture, it was possible to generate an acetate salt of said metal capable of catalyzing the depolymerization reaction with an efficiency that was fully comparable, both in terms of reaction rate and conversion yield of PET to BHET, to that of the same compounds prepared ex *situ* and added to the reaction mixture.

The formation of the catalyst *in situ* from reagents with a low environmental impact, which are readily available and low cost, makes it possible to overcome the disadvantages associated with the supply of the catalysts of the known art referred to above.

It has also been observed that, advantageously, the catalyst can be prepared *in situ* by using as metal compounds the compounds present as contaminants in the material comprising PET supplied to depolymerization, such as calcium carbonate, mixed calcium and magnesium carbonates or aluminium hydroxide commonly present in the material derived from post-consumer carpet waste. This makes it possible to exploit a wider range of PET waste for the production of BHET, in particular those that are currently mainly disposed of in landfill, such as post-consumer carpet waste.

In addition, the *in situ* catalyst preparation method described here is not to be limited only to acetate salts of Ca, Mg and Al. Indeed, it can be advantageously exploited in the depolymerization of a material comprising PET by glycolysis in the presence of any metal acetate salt capable of effectively acting as a depolymerization catalyst, such as catalysts of the known art like zinc acetate, lead acetate, iron acetate, manganese acetate and antimony acetate.

Thus, in this description is first disclosed a process for depolymerizing a material comprising polyethylene terephthalate (PET) by glycolysis with monoethylene glycol (EG), comprising reacting the material comprising PET with EG in the presence of at least one catalyst so as to obtain a glycolized product containing bis(2-hydroxyethyl) terephthalate (BHET) and/or oligomers thereof, wherein said catalyst comprises an acetate salt of a metal chosen from: calcium, magnesium, aluminium and mixtures thereof.

The present invention relates to a process for depolymerizing a material comprising polyethylene terephthalate (PET) by glycolysis with ethylene glycol (EG), comprising reacting PET with EG in the presence of at least one catalyst comprising an acetate of a metal so as to obtain a glycolized product containing bis(2-hydroxyethyl) terephthalate (BHET) and/or oligomers thereof, wherein the process comprises a step of preparing said catalyst by in situ reaction between acetic acid and a compound containing said metal.

In general, the material comprising PET supplied to the depolymerization process according to the present invention may be any material comprising a PET, preferably in an amount greater than or equal to 60% by weight, more preferably greater than or equal to 70% by weight, even more preferably greater than or equal to 80% by weight. The material may comprise contaminants, i.e. compounds other than PET, in an amount of up to 40% by weight, preferably in an amount of up to 30% by weight, even more preferably in an amount of up to 200 by weight.

Examples of contaminants typically present in material including PET from recovered waste are: dyes, polyolefins (e.g. polyethylene or polypropylene), rubber latex, polyamides, polyvinyl alcohol (EVOH), polyvinyl acetate (EVA), UV absorbers; fillers (e.g. calcium carbonate, magnesium carbonate, aluminium hydroxide, titanium dioxide, carbon black, silica), metal particles (e.g. aluminium) and other contaminants deriving from the use of the article.

Preferably, the material comprising PET comprises post-consumer and/or post-industrial PET waste, which may derive, for example, from a wide variety of products such as:
- transparent and/or coloured PET bottles for food products (e.g. water, soft drinks and the like);
- multilayer PET articles, typically for the food industry, in which PET layers are coupled with layers of other polymeric or metallic materials;
- articles containing PET fibers, such as textiles, carpets and the like.

In a particularly preferred embodiment, the depolymerized material comprises post-consumer wastes and/or post-industrial scraps containing PET, preferably post-consumer carpet wastes containing PET.

The material comprising PET can be fed to the depolymerization process in the form of granules, flakes, fibers or fluff.

The depolymerization catalyst is present in the reaction mixture in an amount effective for depolymerizing the PET present therein. Preferably, the catalyst is present in the reaction mixture in an amount in the range of 0.1% to 5% by weight, more preferably in the range of 0.4% to 2.0% by weight with respect to the weight of PET contained in the material undergoing depolymerization.

Preferably, the catalyst is present in the reaction mixture in a ratio of mmol catalyst/kg (PET + EG) greater than 5, more preferably greater than 15, even more preferably greater than 20, for example in the range of 20 to 50, wherein "PET + EG" represents the total weight of EG and PET present in the material comprising PET.

As mentioned above, the catalyst can be prepared ex *situ* and added as such to the reaction mixture, or it can be prepared *in situ* by reaction of acetic acid and a compound containing at least one metal capable of forming an acetate salt of the metal with the acetic acid.

In this description, the expression "acetate salt of the metal" also includes mixed salts, i.e. salts of metal in which, in addition to the acetate anion (hereinafter also referred to as "Ac"), one or more counter-ions other than the acetate ion are present. An example of a mixed salt is calcium bicarbonate acetate, also referred to below as Ca(HCO₃)Ac, which can be obtained from the reaction between acetic acid and calcium carbonate in appropriate stoichiometric ratios.

Preferably, the compound containing the metal is selected from: oxide, hydroxide, carbonate, bicarbonate, chloride and mixtures thereof.

Preferably, the compound containing the metal is selected from: calcium carbonate, calcium bicarbonate, calcium hydroxide, magnesium carbonate, magnesium bicarbonate, magnesium hydroxide, aluminum hydroxide, aluminum trichloride.

As mentioned above, when the catalyst is prepared *in situ,* it may include acetate salts other than Ca, Mg and Al acetates. In such a case, the compound containing the metal comprises at least one metal selected from: zinc, iron, manganese, antimony and mixtures thereof; preferably, the aforesaid compound is in the form of an oxide, hydroxide, carbonate, bicarbonate or chloride.

The reagents required to form the *in situ* catalyst can be added to the EG, or to the mixture containing EG and the material comprising PET, together or separately.

In a preferred embodiment, the material comprising PET further comprises the compound containing the metal in a catalytically effective amount, i.e. an amount sufficient to provide by reaction with acetic acid an acetate salt capable of catalyzing the depolymerization reaction. If the concentration of the compound containing the metal is not sufficient to obtain the metal acetate in a catalytically effective quantity, the metal compound can be added to the reaction mixture on purpose.

Preferably the acetic acid is added to the reaction mixture in an amount in the range of 0.01% - 5% by weight with respect to the material comprising PET, more preferably in the range of 0.03% - 1.5% by weight, even more preferably in the range of 0.1% - 1% by weight.

Preferably the compound containing the metal is added to the reaction mixture in an amount in the range of 0.01% - 5% by weight with respect to the material comprising PET, more preferably in the range of 0.02% - 1.8% by weight, even more preferably in the range of 0.06% - 1.2% by weight.

Preferably the molar ratio between acetic acid and metal compound is in the range of 1:1 to 1:10.

The Applicant noted that the metal acetate formation reaction may compete with the reaction between acetic acid and EG with formation of the corresponding ethylene glycol mono- (EGMA) or di-acetate polyester (EGDA), thus affecting the yield of the acetate salt catalyst formation reaction. However, it has been found that this can be overcome by adding acetic acid and the metal compound to the EG or to the mixture of EG and PET under appropriate conditions. In particular, it is preferable to proceed by first mixing the acetic acid and the metal compound with the EG, and possibly the material containing PET, at a relatively low temperature, preferably in the range of 20°C to 70°C, more preferably 20°C to 50°C, even more preferably 20°C to 40°C, and then raising the temperature of the mixture to the temperature selected for the depolymerization reaction. Without referring to any particular theory, it is believed that at low temperatures the catalyst formation reaction is favored over the formation of the EGMA and EGDA esters; once the metal acetate is formed, the temperature of the reaction mixture can be raised to the depolymerization temperature without this resulting in the formation of the EGMA and EGDA esters.

In case the metal compound is contained in the material comprising PET to be depolymerized, the latter is also added to the EG and acetic acid preferably at a low temperature.

Specifically, in one embodiment of the present invention said step of preparing said catalyst by in situ reaction comprises:
a. mixing acetic acid, the compound containing the metal and EG at a temperature within the range from 20°C to 70°C, preferably from 20°C to 50°C;
b. heating the mixture obtained in step a up to a temperature within the range from 170°C to 230°C;
c. feeding the material comprising PET to the mixture obtained in step b to obtain the glycolyzed product.

Preparation of the catalyst *in situ* is particularly useful where the material comprising PET also contains one or more metal compounds that act as precursors for the *in situ* formation of the catalyst by reaction with acetic acid. In practice, it is possible to determine the metal content of the material and consequently dose the acetic acid in the reaction mixture. If the metal compound is present in the material comprising PET in a relatively large quantity, it is always possible to dose enough acetic acid to transform only part of the metal compound and generate the necessary amount of catalyst. The remaining part of the metal compound present as a contaminant is then removed in the purification step of the glycolized product. This is particularly the case for recycled PET from recovered carpets *(fluff*), which typically contains at least 3-5% calcium carbonate and/or aluminium hydroxide by weight.

Specifically, in one embodiment of the present invention said step of preparing said catalyst by in situ reaction comprises:
a. mixing EG, acetic acid and the material comprising PET and the compound containing said metal at a temperature within the range 20°C to 70°C, preferably 20°C - 50°C;
b. heating the mixture obtained in step a up to a temperature within the range 170°C - 230°C and make the reaction take place to obtain the glycolyzed product.

Preferably, the glycolysis reaction is performed at a temperature in the range of 170°C to 230°C, more preferably in the range of 190°C to 210°C. The heating of the reaction mixture is not carried out by microwave irradiation.

In one embodiment, the heating of the reaction mixture is not carried out by microwave irradiation when the catalyst comprises magnesium acetate.

In the glycolysis reaction, preferably, the EG is used in a weight ratio of EG:PETm in the range of 1:1 to 8:1, more preferably in the range of 1:1 to 4:1, even more preferably in the range of 1:1 to 2:1, wherein PETm denotes the material comprising PET.

The duration of the glycolysis reaction can vary within wide ranges, depending on the reaction conditions, such as temperature, agitation, type of reactor and the like. Usually the reaction time is 1 to 6 hours, preferably 1.5 to 4 hours. The reaction can be carried out in a batch or continuous process. The reaction pressure is usually atmospheric pressure, but reduced or increased pressure may be used.

The glycolysis reaction leads to the formation of a glycolyzed product comprising BHET and/or oligomers thereof. At the end of depolymerization, typically the glycolized product also contains a residual fraction of the material comprising unreacted PET and other depolymerization products (e.g. water, contaminants insoluble in EG, etc.).

BHET and/or oligomers thereof can be separated from the glycolized product and purified using suitable techniques of the known art such as distillation, filtration, crystallization and the like.

BHET and/or oligomers thereof obtained by the process described herein can be used as raw materials in new production processes for polyester polymers and copolymers. For example, they can be polymerized to produce PET exclusively from recycled BHET or in combination with BHET made from virgin raw materials.

The process described herein can be carried out with the devices and equipment known in the art for the production of polymers, in particular polyesters.

Advantageously, the depolymerization reaction can be conducted in an acid-resistant stainless steel reactor equipped with suitable inlets for dosing liquid acetic acid and solid compounds for *in situ* catalyst formation. The reactor can be equipped with heating and mixing means to heat the reaction mixture to the desired temperature and keep it stirred.

The following examples are provided purely for the purpose of illustration of the present invention and should not be regarded as limiting the scope of protection defined by the appended claims.

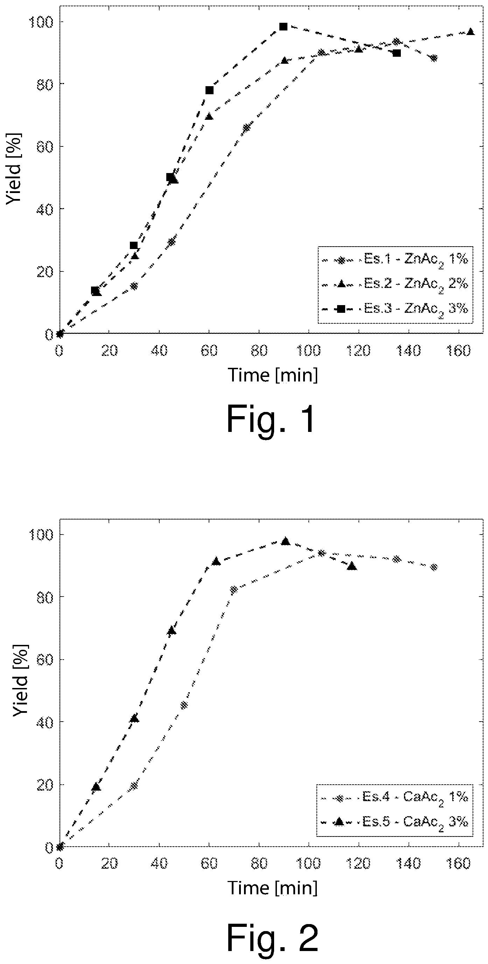

In the examples, reference is made to the attached Figures 1 to 7, which graphically show the time-dependent trend of the total yield of the glycolysis reaction of examples 1 to 14.

### EXAMPLES

### 1. Depolymerization test

The effectiveness of the process according to the present description has been verified through a series of catalytic depolymerization tests of different types of waste containing PET. The depolymerization test was conducted as follows.

The glycolysis reaction was carried out in a 1 liter round bottomed Pyrex glass reaction flask. The flask is heated via a heating mantle (isomantle). The reaction flask has four necks with the following functions:
- a thermocouple is inserted into the first neck to detect the temperature of the liquid mixture during the reaction: the temperature set point can be set digitally on the heating mantle;
- a reflux condenser is inserted into the second neck to condense the vapors developed during the reaction by means of a continuously fed stream of cooling water;
- the remaining two necks are used to feed the monoethylene glycol (EG) and the material comprising PET; during the depolymerization reaction, these two necks are kept closed by means of two frosted glass stoppers.

The reaction mixture was kept under stirring by means of a magnetic stirrer with the aid of a PTFE magnetic stirring rod, the rotation speed of which can be digitally set on the isomantle. The reaction temperature and rotation speed were set at 195°C and 1400 rpm respectively.

The EG and catalyst (if prepared ex *situ*) are first loaded into the reaction flask. The mixture, kept under stirring, is then heated to reaction temperature, recondensing the vapors developed through the bubble condenser. Once the mixture of glycol and catalyst has reached the reaction temperature, the material comprising PET (hereinafter also referred to as "PET" only), which has been preheated to 110°C, is introduced into the flask.

The moment when loading of the PET into the reaction flask begins is considered to be the reaction start "time 0" (t₀). Starting at t₀, a sample of the glycolysis fluid is taken at regular intervals using a glass Pasteur pipette; the liquid is then transferred to a vial cooled with cold water to stop the reaction progressing and then analysed by HPLC to determine the content of BHET and its oligomers.

The time in which complete dissolution of PET in glycol is observed is also noted.

At the end of glycolysis, the contents of the flask are discharged and the liquid is subjected to end-of-process HPLC analysis.

### 2. Examples 1-3 (comparative) - catalyst: zinc acetate (ZnAc₂)

Following the procedure described in point 1, the depolymerization of granulated PET was carried out in the presence of zinc acetate, dosing the catalyst at different concentrations. The PET used was in a substantially pure form (approx. 100% purity). The composition of the reaction mixtures is provided in Tables 1 - 3 together with the results of the analysis of the reaction liquid taken at different reaction times.

The weight ratios EG/PET and catalyst/PET refer to the weight ratios of EG and catalyst, respectively, to the weight of the material including PET.

The parameters "BHET yield", "dimer yield" (of BHET) and "total yield" refer to the ratios, respectively, of the amounts of BHET, dimer of BHT and sum "BHET + dimer" referring to the weight of the material comprising PET, expressed as a mole/mole percentage.

**Table 1**

| Example | PET [g] | PET purity [%] | EG [g] | EG/PET ratio | Catalyst type | Catal. [g] | Catal./PET [%] | T [°C] | Dissolution time [min] |
|---|---|---|---|---|---|---|---|---|---|
| 1 | 30 | 100 | 120 | 4 | ZnAc₂ | 0.3 | 1 | 195 | 135 |

| Sample analysis | | | | | | | | | |
|---|---|---|---|---|---|---|---|---|---|
| Time [min] | | 0 | 30 | 45 | 75 | 105 | 135 | 150 | |
| BHET yield [%] | | 0 | 14.68 | 26.96 | 62.25 | 82.14 | 84.66 | 79.67 | |
| Dimer yield [%] | | 0 | 0.28 | 1.17 | 1.82 | 3.96 | 4.44 | 4.24 | |
| Total yield [%] | | 0 | 15.24 | 29.30 | 65.89 | 90.06 | 93.54 | 88.15 | |

**Table 2**

| Example | PET [g] | PET purity [%] | EG [g] | EG/PET ratio | Catalyst type | Catal. [g] | Catal./PET [%] | T [°C] | Dissolution time [min] |
|---|---|---|---|---|---|---|---|---|---|
| 2 | 30 | 100 | 120 | 4 | ZnAc₂ | 0.6 | 2 | 195 | 90 |

| Sample analysis | | | | | | | | | |
|---|---|---|---|---|---|---|---|---|---|
| Time [min] | | 0 | 15 | 30 | 60 | 90 | 120 | 165 | |
| BHET yield [%] | | 0 | 13.82 | 25.53 | 64.84 | 79.22 | 82.24 | 87.45 | |
| Dimer yield [%] | | 0 | 0.10 | 1.12 | 2.57 | 4.08 | 4.34 | 4.62 | |
| Total yield [%] | | 0 | 14.02 | 27.77 | 69.98 | 87.38 | 90.92 | 96.69 | |

**Table 3**

| Example | PET [g] | PET purity [%] | EG [g] | EG/PET ratio | Catalyst type | Catal. [g] | Catal./PET [%] | T [°C] | Dissolution time [min] |
|---|---|---|---|---|---|---|---|---|---|
| 3 | 30 | 100 | 120 | 4 | ZnAc₂ | 0.9 | 3 | 195 | 90 |

| Sample analysis | | | | | | | | | |
|---|---|---|---|---|---|---|---|---|---|
| Time [min] | | 0 | 15 | 30 | 45 | 60 | 90 | 135 | |
| BHET yield [%] | | 0 | 12.34 | 22.38 | 47.37 | 73.69 | 91.89 | 81.23 | |
| Dimer yield [%] | | 0 | 0.40 | 0.86 | 1.30 | 2.40 | 3.52 | 4.41 | |
| Total yield [%] | | 0 | 13.14 | 24.10 | 49.97 | 78.49 | 98.93 | 90.05 | |

Figure 1 shows the time-dependent trend of the total yield of the depolymerization reaction.

### 3. Reference Examples 4-5 - catalyst: calcium acetate (CaAc₂)

Following the process described in point 1, a PET (100% purity) in granules was depolymerized in the presence of calcium acetate dosed at 1% and 3% by weight of the loaded PET.

The composition of the reaction mixtures is provided in Tables 4 - 5 together with the results of the analysis of the reaction liquid taken at different reaction times.

**Table 4**

| Example | PET [g] | PET purity [%] | EG [g] | EG/PET ratio | Catalyst type | Catal. [g] | Catal./PET [%] | T [°C] | Dissolution time [min] |
|---|---|---|---|---|---|---|---|---|---|
| 4 | 30 | 100 | 120 | 4 | CaAc₂ | 0.3 | 1 | 195 | 120 |

| Sample analysis | | | | | | | | | |
|---|---|---|---|---|---|---|---|---|---|
| Time [min] | | 0 | 30 | 50 | 70 | 105 | 135 | 150 | |
| BHET yield [%] | | 0 | 18.76 | 43.29 | 74.21 | 84.85 | 84.24 | 82.04 | |
| Dimer yield [%] | | 0 | 0.35 | 1.06 | 4.01 | 4.55 | 3.88 | 3.70 | |
| Total yield [%] | | 0 | 19.46 | 45.41 | 82.23 | 93.95 | 92.00 | 89.44 | |

**Table 5**

| Example | PET [g] | PET purity [%] | EG [g] | EG/PET ratio | Catalyst type | Catal. [g] | Catal./PET [%] | T [°C] | Dissolution time [min] |
|---|---|---|---|---|---|---|---|---|---|
| 5 | 30 | 100 | 120 | 4 | CaAc₂ | 0.9 | 3 | 195 | 90 |

| Sample analysis | | | | | | | | | |
|---|---|---|---|---|---|---|---|---|---|
| Time [min] | | 0 | 15 | 30 | 45 | 60 | 90 | 120 | |
| BHET yield [%] | | 0 | 18.39 | 38.99 | 65.74 | 85.44 | 94.21 | 83.44 | |
| Dimer yield [%] | | 0 | 0.49 | 0.90 | 1.56 | 2.38 | 2.00 | 2.84 | |
| Total yield [%] | | 0 | 19.37 | 40.79 | 68.86 | 90.20 | 98.21 | 89.12 | |

Figure 2 shows the time-dependent trend of the total yield of the depolymerization reaction.

Comparison of the results of examples 1 -3 and 4 -5 shows that the CaAc2 catalyst has an efficiency comparable to that of ZnAc2 and faster depolymerization kinetics than ZnAc2 with the same weight of catalyst.

### 4. Reference Examples 6-7

Since the physical form of the material comprising PET can also influence the extent of the depolymerization reaction, the procedure described in step 1 was carried out by feeding a waste comprising PET consisting of spinning waste (purity approx. 97.5%) and using ZnAc₂ or CaAc₂ as a catalyst.

The composition of the reaction mixtures is provided in Tables 6 - 7 together with the results of the analysis of the reaction liquid taken at different reaction times.

**Table 6**

| Example | PET [g] | PET purity [%] | EG [g] | EG/PET ratio | Catalyst type | Catal. [g] | Catal./PET [%] | T [°C] | Dissolution time [min] |
|---|---|---|---|---|---|---|---|---|---|
| 6 | 30 | 97.5 | 120 | 4 | ZnAc₂ | 1.2 | 4 | 195 | 15 |

| Sample analysis | | | | | | | | | |
|---|---|---|---|---|---|---|---|---|---|
| Time [min] | | 0 | 10 | 20 | 30 | 60 | 90 | 120 | 180 |
| BHET yield [%] | | 0 | 21.0 | 63.58 | 75.57 | 71.3 | 76.76 | 76.97 | 84.50 |
| Dimer yield [%] | | 0 | 2.4 | 3.95 | 4.12 | 3.87 | 3.57 | 3.89 | 4.22 |
| Total yield [%] | | 0 | 25.80 | 71.48 | 83.81 | 79.04 | 83.90 | 84.75 | 92.94 |

**Table 7**

| Example | PET [g] | PET purity [%] | EG [g] | EG/PET ratio | Catalyst type | Catal. [g] | Catal./PET [%] | T [°C] | Dissolution time [min] |
|---|---|---|---|---|---|---|---|---|---|
| 7 | 30 | 97.5 | 120 | 4 | CaAc₂ | 0.9 | 3 | 195 | 10 |

| Sample analysis | | | | | | | | | |
|---|---|---|---|---|---|---|---|---|---|
| Time [min] | | 0 | 5 | 12 | 22 | 32 | 72 | 122 | |
| BHET yield [%] | | 0 | 62.53 | 80.39 | 80.64 | 80.42 | 75.98 | 81.84 | |
| Dimer yield [%] | | 0 | 3.90 | 4.09 | 4.26 | 4.20 | 3.83 | 3.87 | |
| Total yield [%] | | 0 | 70.33 | 88.57 | 89.16 | 88.82 | 83.64 | 89.58 | |

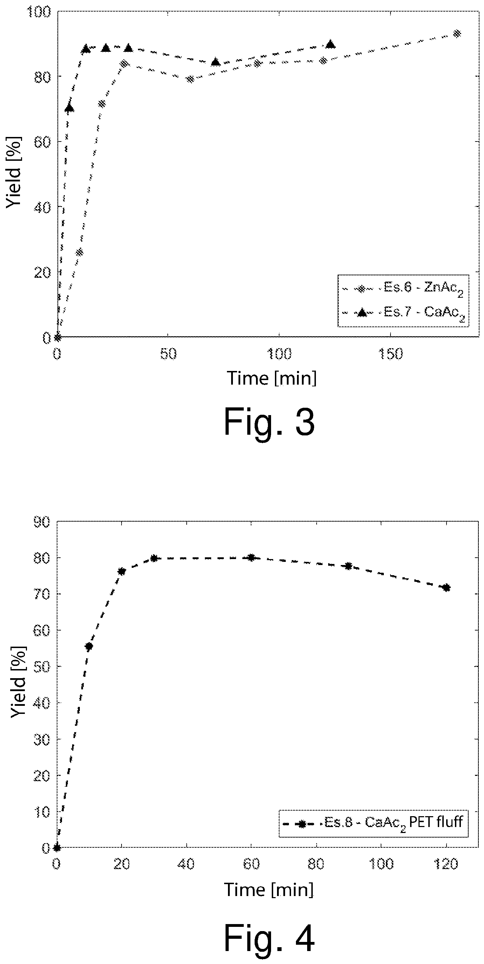

Figure 3 shows the time-dependent trend of the total yield of the depolymerization reaction of Examples 6 and 7.

It can be seen that two different weight dosages are compared in these examples, but they correspond to the same mole dosage (approx. 5.5 mmol of catalyst).

The graphical comparison in Figure 3 shows that CaAc₂ is a depolymerization catalyst that achieves results quite comparable to those of conventional ZnAc₂ even at lower weight concentrations. The reaction in the presence of CaAc₂ is completed more quickly than with ZnAc₂.

### 5. Reference Examples 8-10 - catalyst: calcium acetate (CaAc₂)

The effectiveness of the CaAc₂ catalyst was tested in the depolymerization reaction of a PET waste from carpet recovery (fluff) . The fluff used had a PET content of 80% by weight.

The process described in point 1 was carried out by feeding the fluff into a reaction mixture including CaAc₂ as a catalyst.

In the tests in which fluff was fed, it was observed that, at the end of the glycolysis reaction, the reaction mixture at 195°C still had a fraction of suspended solids with a yellowish-brown appearance, which was removed by hot filtration (approx. 190°C) of the reaction mixture. The insoluble residue separated by filtration, amounting to about 10-15% of the weight of the loaded fluff, was analysed by DSC, TGA and FT-IR. Analyses revealed the presence of a mixture of polypropylene, glues and other inorganic substances, including calcium carbonate.

The composition of the reaction mixtures is provided in Tables 8 - 10 together with the results of the analysis of the reaction liquid taken at different reaction times.

**Table 8**

| Example | PET [g] | PET purity [%] | EG [g] | EG/PET ratio | Catalyst type | Catal. [g] | Catal./PET [%] | T [°C] | Dissolution time [min] |
|---|---|---|---|---|---|---|---|---|---|
| 8 | 60 | 80 | 240 | 4 | CaAc₂ | 1.05 | 1.75 | 195 | 10 |

| Sample analysis | | | | | | | | | |
|---|---|---|---|---|---|---|---|---|---|
| Time [min] | | 0 | 10 | 20 | 30 | 60 | 90 | 120 | |
| BHET yield [%] | | 0 | 50.95 | 70.06 | 73.44 | 73.43 | 71.34 | 66.05 | |
| Dimer yield [%] | | 0 | 2.24 | 3.02 | 3.10 | 3.20 | 3.06 | 2.78 | |
| Total yield [%] | | 0 | 55.43 | 76.10 | 79.63 | 79.83 | 77.46 | 71.60 | |

**Table 9**

| Example | PET [g] | PET purity [%] | EG [g] | EG/PET ratio | Cataly st type | Catal. [g] | Catal. /PET [%] | T [°C] | Dissolut ion time [min] | |
|---|---|---|---|---|---|---|---|---|---|---|
| 9 | 100 | 80 | 200 | 2 | CaAc₂ | 0.35 | 0.35 | 195 | 15 | |

| Sample analysis | | | | | | | | | | |
|---|---|---|---|---|---|---|---|---|---|---|
| Time [min] | | 0 | 10 | 22 | 30 | 45 | 60 | 90 | 120 | 150 |
| BHET yield [%] | | 0 | 15.03 | 32.94 | 39.34 | 51.77 | 53.19 | 54.22 | 54.22 | 53.69 |
| Dimer yield [%] | | 0 | 0.94 | 2.66 | 3.17 | 4.50 | 4.21 | 4.36 | 4.55 | 4.58 |
| Total yield [%] | | 0 | 16.92 | 38.26 | 45.68 | 60.78 | 61.61 | 62.94 | 63.32 | 62.84 |

**Table 10**

| Example | PET [g] | PET purity [%] | EG [g] | EG/PET ratio | Catalyst type | Catal. [g] | Catal./PET [%] | T [°C] | Dissolution time [min] |
|---|---|---|---|---|---|---|---|---|---|
| 10 | 100 | 80 | 200 | 2 | CaAc₂ | 0.18 | 0.18 | 195 | 20 |

| Sample analysis | | | | | | | | | |
|---|---|---|---|---|---|---|---|---|---|
| Time [min] | | 0 | 10 | 21 | 30 | 45 | 90 | 120 | |
| BHET yield [%] | | 0 | 9.02 | 17.00 | 26.23 | 49.97 | 55.65 | 49.58 | |
| Dimer yield [%] | | 0 | 0.34 | 0.66 | 0.70 | 2.12 | 3.78 | 3.15 | |
| Total yield [%] | | 0 | 9.70 | 18.31 | 27.62 | 54.21 | 63.22 | 55.89 | |

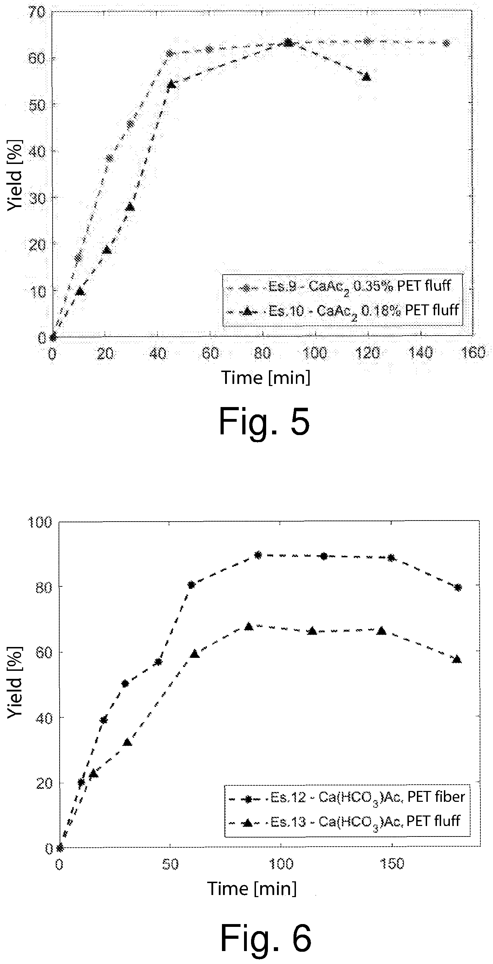

Figure 4 shows the time-dependent trend of the total yield of the depolymerization reaction in Example 8, while Figure 5 shows that of the reactions in Examples 9-10.

Examples 9 and 10 show that even when operating with relatively low CaAc₂ catalyst concentrations and EG/PET ratios, conversion yields of PET to BHET and oligomers comparable to those of catalysts used in glycolysis processes of the known art, such as ZnAc₂, are obtained at higher EG/PET ratios.

### 6. Inventive Examples 11-12 - catalysts: acetic acid and calcium bicarbonate acetate (Ca(HCO₃)Ac) generated in situ

Following the procedure described in point 1, the following depolymerization tests were performed. In Example 11 (comparative), the depolymerization test was carried out using PET in substantially contaminant-free fiber form (purity approx. 97.5%). Only acetic acid was added to the reaction mixture to test its possible effect as a depolymerization catalyst. Acetic acid was added to the EG at 30°C, then the mixture was heated to 195°C, keeping it under reflux. At this temperature, the PET fiber was added. After 3 hours of reaction, no dissolution of the fiber was observed and HPLC analysis of the reaction mixture liquid did not detect the presence of BHET.

Example 12 was carried out under the same conditions as Example 11, but by adding equimolar amounts of CaCOs and acetic acid to the EG at 30°C. The mixture was then heated to 195°C, keeping it under reflux, and PET fiber was added. The reaction continued until the fiber was completely dissolved. The data shown in Table 11 thus demonstrate that an active stoichiometric catalyst Ca(HCO₃)Ac was generated in the reaction mixture at a concentration of 2.66% by weight with respect to the weight of the PET fiber.

**Table 11**

| Example | PET [g] | PET purity [%] | EG [g] | EG/PET ratio | Catalyst type | Catal. [g] | Catal. /PET [%] | T [°C] | Dissolution time [min] |
|---|---|---|---|---|---|---|---|---|---|
| 12 | 30 | 97.5 | 120 | 4 | Ca(HCO₃)Ac | 0.8 | 2.66 | 195 | 50 |

| Sample analysis | | | | | | | | | |
|---|---|---|---|---|---|---|---|---|---|
| Time [min] | | 0 | 10 | 20 | 30 | 45 | 60 | 90 | 120 |
| BHET yield [%] | | 0 | 17.03 | 33.64 | 44.40 | 50.18 | 72.39 | 80.90 | 81.11 |
| Dimer yield [%] | | 0 | 1.59 | 2.74 | 2.88 | 3.28 | 4.09 | 4.27 | 3.97 |
| Total yield [%] | | 0 | 20.21 | 39.12 | 50.16 | 56.74 | 80.57 | 89.44 | 89.05 |
| | | Time [min] | | 150 | 180 | | | | |
| | | BHET yield [%] | | 80.89 | 72.52 | | | | |
| | | Dimer yield [%] | | 3.86 | 3.39 | | | | |
| | | Total yield [%] | | 88.61 | 79.30 | | | | |

Figure 6 shows the time-dependent trend of the total yield of the depolymerization reaction of Example 12 in comparison with that of Example 13.

### 7. Inventive Example 13 - catalysts: acetic acid and calcium bicarbonate acetate (Ca(HCO₃)Ac) generated in situ

Example 13 was carried out under the same conditions as Example 12, but by adding acetic acid and PET fluff to the EG at 30°C and using the CaCOs contained in the PET fluff (80% purity). The CaCOs content in the fluff was approx. 5% by weight with respect to the weight of the fluff.

The composition of the reaction mixtures is provided in Table 12 together with the results of the analysis of the reaction liquid taken at different reaction times.

**Table 12**

| Example | PET [g] | PET purity [%] | EG [g] | EG/PET ratio | Catalyst type | Catal. [g] | Catal. /PET [%] | T [°C] | Dissolution time [min] |
|---|---|---|---|---|---|---|---|---|---|
| 13 | 30 | 80 | 120 | 4 | Ca(HCO₃)Ac | 0.45 | 1.5 | 195 | 50 |

| Sample analysis | | | | | | | | | |
|---|---|---|---|---|---|---|---|---|---|
| Time [min] | | 0 | 15 | 30 | 60 | 85 | 115 | 145 | 180 |
| BHET yield [%] | 0 | 19.37 | 28.06 | 53.30 | 61.74 | 59.33 | 60.70 | 51.68 | |
| Dimer yield [%] | 0 | 1.70 | 1.46 | 2.70 | 3.19 | 3.26 | 2.94 | 2.80 | |
| Total yield [%] | 0 | 22.76 | 30.99 | 58.71 | 68.13 | 65.84 | 66.58 | 57.28 | |

Figure 6 shows the time-dependent trend of the total yield of the depolymerization reaction of Example 13. The results show that the PET glycolysis process of Example 13 occurs to a similar extent as that of Example 12, confirming that it is possible to prepare *in situ* an effective depolymerization catalyst by exploiting the metal compounds present as contaminants in the material comprising recycled PET.

### 8. Reference Example 14 - catalyst: magnesium acetate (MgAc₂)

Following the process described in point 1, a PET (100% purity) in granules was depolymerized using magnesium acetate dosed at 0.45% by weight of MgAc₂ with respect to the weight of the loaded PET.

The composition of the reaction mixtures is provided in Table 13 together with the results of the analysis of the reaction liquid taken at different reaction times.

**Table 13**

| Example | PET [g] | PET purity [%] | EG [g] | EG/PET ratio | Catalyst type | Catal. [g] | Catal. /PET [%] | T [°C] | Dissolution time [min] |
|---|---|---|---|---|---|---|---|---|---|
| 14 | 100 | 100 | 200 | 2 | MgAc₂ | 0.45 | 0.45 | 195 | 130 |

| Sample analysis | | | | | | | | | |
|---|---|---|---|---|---|---|---|---|---|
| Time [min] | | 0 | 10 | 20 | 30 | 45 | 60 | 90 | 120 |
| BHET yield [%] | | 0 | 11.99 | 32.10 | 35.23 | 50.80 | 61.02 | 60.02 | 64.10 |
| Dimer yield [%] | | 0 | 0.72 | 1.87 | 2.34 | 3.65 | 4.93 | 5.20 | 5.47 |
| Total yield [%] | | 0 | 13.43 | 35.84 | 39.91 | 58.1 | 70.88 | 70.42 | 75.04 |
| | | Time [min] | | 150 | 180 | | | | |
| | | BHET yield [%] | | 65.14 | 66.27 | | | | |
| Dimer yield [%] | 5.45 | 5.78 | | | | | | | |
| Total yield [%] | 76.04 | 77.83 | | | | | | | |

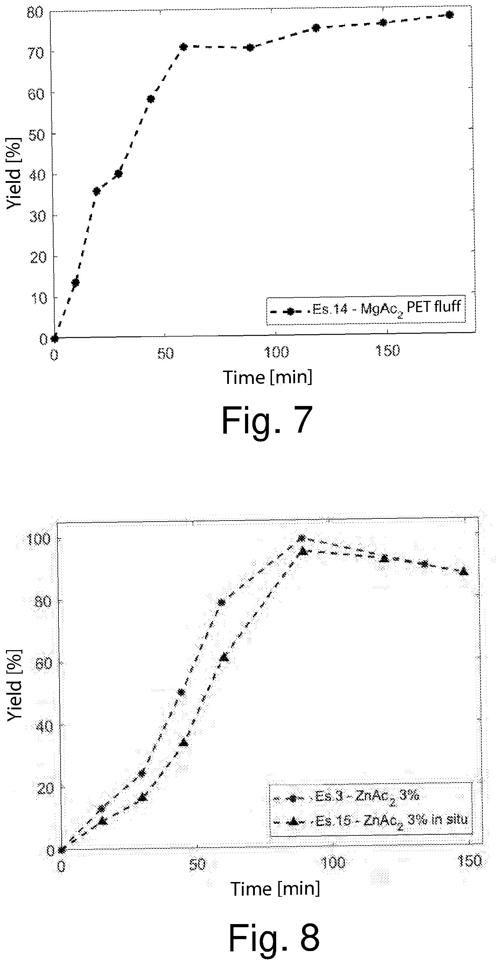

Figure 7 shows the time-dependent trend of the total yield of the depolymerization reaction of Example 14. The results show that magnesium acetate is also an effective catalyst in PET depolymerization.

### 9. Inventive Examples 15-18 - Other catalysts generated in situ

Examples 15 to 18 were carried out under the same conditions as Example 12, adding to EG at 30°C respectively:
- in Example 15, acetic acid and zinc carbonate in a 2:1 molar ratio;
- in Example 16, acetic acid and calcium oxide in a 2:1 molar ratio;
- in Example 17, acetic acid and magnesium hydroxide in a 2:1 molar ratio;
- in Example 18, acetic acid and aluminium hydroxide in a molar ratio of 3:1.

In all cases the mixture was then heated to 195°C, keeping it under reflux, and PET granules were added.

The composition of the reaction mixtures is provided in Tables 14 to 17, together with the results of the analysis of the reaction liquid taken at different reaction times.

**Table 14**

| Example | PET [g] | PET purity [%] | EG [g] | EG/PET ratio | Catalyst type | Catal. [g] | Catal./PET [%] | T [°C] | Dissolution time [min] |
|---|---|---|---|---|---|---|---|---|---|
| 15 | 30 | 100 | 120 | 4 | ZnAc₂ | 0.9 | 3 | 195 | 120 |

| Sample analysis | | | | | | | | | |
|---|---|---|---|---|---|---|---|---|---|
| Time [min] | | 0 | 15 | 30 | 45 | 60 | 90 | 120 | 150 |
| BHET yield [%] | | 0 | 8.14 | 14.47 | 30.33 | 55.12 | 86.34 | 81.92 | 77.08 |
| Dimer yield [%] | | 0 | 0.37 | 0.65 | 1.19 | 2.53 | 4.33 | 5.19 | 5.16 |
| Total yield [%] | | 0 | 8.88 | 15.77 | 32.71 | 60.18 | 95.00 | 92.30 | 87.40 |

**Table 15**

| Example | PET [g] | PET purity [%] | EG [g] | EG/PET ratio | Catalyst type | Catal. [g] | Catal./PET [%] | T [°C] | Dissolution time [min] |
|---|---|---|---|---|---|---|---|---|---|
| 16 | 30 | 100 | 120 | 4 | CaAc₂ | 0.9 | 3 | 195 | 115 |

| Sample analysis | | | | | | | | | |
|---|---|---|---|---|---|---|---|---|---|
| Time [min] | | 0 | 15 | 30 | 45 | 60 | 90 | 120 | 150 |
| BHET yield [%] | | 0 | 10.72 | 17.37 | 36.73 | 63.35 | 85.06 | 83.25 | 82.61 |
| Dimer yield [%] | | 0 | 0.54 | 0.81 | 1.56 | 2.68 | 3.33 | 4.15 | 3.86 |
| Total yield [%] | | 0 | 11.80 | 18.99 | 39.85 | 68.71 | 91.72 | 91.55 | 90.33 |

**Table 16**

| Example | PET [g] | PET purity [%] | EG [g] | EG/PET ratio | Catalyst type | Catal. [g] | Catal./PET [%] | T [°C] | Dissolution time [min] |
|---|---|---|---|---|---|---|---|---|---|
| 17 | 60 | 100 | 120 | 2 | MgAc₂ | 1.2 | 2 | 195 | 130 |

| Sample analysis | | | | | | | | | |
|---|---|---|---|---|---|---|---|---|---|
| Time [min] | | 0 | 15 | 30 | 45 | 60 | 75 | 90 | 120 |
| BHET yield [%] | | 0 | 3.51 | 9.86 | 10.74 | 34.34 | 46.26 | 58.42 | 66.17 |
| Dimer yield [%] | | 0 | 0.14 | 0.23 | 1.06 | 3.86 | 5.48 | 6.89 | 7.61 |
| Total yield [%] | | 0 | 3.79 | 10.32 | 12.86 | 42.06 | 57.22 | 72.20 | 81.39 |
| | | Time [min] | | 150 | 180 | | | | |
| | | BHET yield [%] | | 69.88 | 78.27 | | | | |
| | | Dimer yield [%] | | 8.73 | 8.93 | | | | |
| | | Total yield [%] | | 87.34 | 96.13 | | | | |

**Table 17**

| Example | PET [g] | PET purity [%] | EG [g] | EG/PET ratio | Catalyst type | Catal. [g] | Catal./PET [%] | T [°C] | Dissolution time [min] | |
|---|---|---|---|---|---|---|---|---|---|---|
| 18 | 60 | 100 | 120 | 2 | AlAc₃ | 1.2 | 2 | 195 | 155 | |

| Sample analysis | | | | | | | | | | |
|---|---|---|---|---|---|---|---|---|---|---|
| Time [min] | | 0 | 15 | 30 | 45 | 60 | 90 | 120 | 150 | 190 |
| BHET yield [%] | | 0 | 5.92 | 11.53 | 18.45 | 22.52 | 44.13 | 50.31 | 52.75 | 56.89 |
| Dimer yield [%] | | 0 | 0.15 | 0.29 | 1.25 | 2.55 | 4.89 | 5.23 | 5.77 | 6.22 |
| Total yield [%] | | 0 | 6.22 | 12.11 | 20.95 | 27.62 | 53.91 | 60.77 | 64.29 | 69.33 |

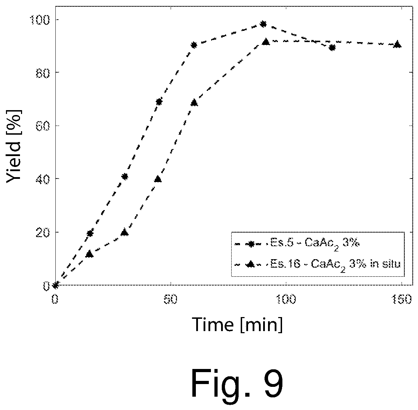

Figure 8 and Figure 9 show the time-dependent trend of the total yield of the depolymerization reaction of Examples 15 and 16, respectively, compared with those of Examples 3 and 5, respectively, where the same catalysts were prepared *ex situ* and then added to the reaction mixture. The results show that the catalysts generated *in situ* are as effective as those produced ex situ.

### 10. Inventive Examples 19-20 - Recovery of BHET by evaporation of EG and crystallization

Using the same process as in Inventive Examples 12 and 13 respectively, the focus in Examples 19 and 20 was on separating BHET and dimer to obtain material that allows polymerization to PET.

The glycolysis residue was filtered to separate unconverted PET and other impurities from the glycolytic material in this first step. Part of the glycol in the permeate is evaporated by flash evaporation under vacuum.

At this point, a large excess of water (4 times the weight of the glycol) was added to precipitate the product. The latter was then filtered again, washed with water and finally dried.

The resulting material was then analyzed by HPLC, FT-IR, DSC and NMR, confirming that it is BHET and its dimer.

The properties of the separated product (content of COOH groups and diethylene glycol (DEG), melting point) are in line with what is generally required for the materials to be polymerized, as shown in Table 18.

**Table 18**

| | Example 19 | Example 20 |
|---|---|---|
| Starting PET | PET fiber | PET fluff |
| Process parameters | As in Example 12 | As in Example 13 |
| COOH of BHET [mEq/kg] | 36.7 | 35.4 |
| DEG content in BHET [%] | 0.12 | 0.11 |
| Melting point of BHET[°C] | 108.9 | 107.5 |

## Claims

1. Process for depolymerizing a material comprising polyethylene terephthalate (PET) by glycolysis with ethylene glycol (EG), which comprises reacting the material comprising PET with EG in the presence of at least one catalyst comprising a metal acetate so as to obtain a glycolyzed product containing bis(2-idrossietil)terephthalate (BHET) and/or oligomers thereof, wherein the process comprises a step of preparing said catalyst by in situ reaction between acetic acid and a compound containing said metal.

2. Process according to claim 1, wherein said step of preparing said catalyst by in situ reaction between acetic acid and the compound containing said metal comprises:
a. mixing acetic acid, the compound containing said metal and EG at a temperature within the range 20°C - 70°C;
b. heating the mixture obtained in step a up to a temperature within the range 170°C - 230°C;
c. feeding the material comprising PET to the mixture obtained in step b to obtain the glycolyzed product.

3. Process according to claim 2, wherein said step of preparing said catalyst by in situ reaction between acetic acid and the compound containing said metal comprises:
a. mixing EG, acetic acid and the material comprising PET and the compound containing said metal at a temperature within the range 20°C - 70°C;
b. heating the mixture obtained in step a up to a temperature within the range 170°C - 230°C and make the reaction take place to obtain the glycolyzed product.

4. Process according to any one of claims 1 to 3, wherein said metal acetate is selected from: calcium acetate, magnesium acetate, aluminum acetate, zinc acetate, lead acetate, iron acetate, manganese acetate, antimony acetate and mixtures thereof.

5. Process according to any one of claims 1 to 4, wherein the compound containing said metal is selected from: oxide, hydroxide, carbonate, bicarbonate, chloride and mixtures thereof.

6. Process according to any one of claims 1 to 5, wherein the compound containing said metal is selected from: calcium carbonate, calcium bicarbonate, calcium hydroxide, magnesium carbonate, magnesium bicarbonate, magnesium hydroxide, aluminum hydroxide, aluminum trichloride.

7. Process according to any one of claims 1 to 6, wherein the material comprising PET comprises the compound containing said metal.

8. Process according to any one of claims 1 to 7, wherein the material comprising PET comprises post-consumer wastes and/or post-industrial scraps containing PET, preferably post-consumer carpet wastes containing PET.

9. Process according to any one of claims 1 to 8, wherein the material comprising PET is in the form of granules, flakes, fibers or fluff.

10. Process according to any one of claims 1 to 9, wherein the weight ratio EG:PETm is from 1:1 to 8:1, preferably from 1:1 to 4:1, even more preferably from 1:1 to 2:1, wherein PETm indicates the material comprising PET.

## Patentansprüche

1. Verfahren zum Entpolymerisieren eines Materials umfassend Polyethylenterephthalat (PET), durch Glykolyse mit Ethylenglykol (EG), das das Reagieren des PET-umfassenden Materials mit EG in Anwesenheit mindestens eines Metallacetat umfassenden Katalysators, um ein glykolisiertes Produkt zu erhalten, das bis(2-hydroxyethyl)terephthalat (BHET) und/oder Oligomere davon enthält, wobei das Verfahren einen Schritt des Vorbereitens des Katalysators durch eine in-situ-Reaktion zwischen Essigsäure und einer das Metall enthaltenden Verbindung umfasst.

2. Verfahren nach Anspruch 1, wobei der Schritt des Vorbereitens des Katalysators durch eine in-situ-Reaktion zwischen Essigsäure und der das Metall enthaltenden Verbindung umfasst:
a. Mischen Essigsäure, der das Metall enthaltenden Verbindung und EG bei einer Temperatur im Bereich von 20°C bis 70°C;
b. Erhitzen der in Schritt a erhaltenen Mischung auf eine Temperatur im Bereich von 170°C bis 230°C;
c. Zuführen des PET-haltigen Materials zu der in Schritt b erhaltenen Mischung, um das glykolisierte Produkt zu erhalten.

3. Verfahren nach Anspruch 2, wobei der Schritt des Vorbereitens des Katalysators durch eine in-situ-Reaktion zwischen Essigsäure und der das Metall enthaltenden Verbindung umfasst:
a. Mischen EG, Essigsäure und des PET-haltigen Materials und der das Metall enthaltenden Verbindung bei einer Temperatur im Bereich von 20°C bis 70°C;
b. Erhitzen der in Schritt a erhaltenen Mischung auf eine Temperatur im Bereich von 170°C bis 230°C und Durchführen der Reaktion, um das glykolisierte Produkt zu erhalten.

4. Verfahren nach einem der Ansprüche 1 bis 3, wobei das Metallacetat aus: Calciumacetat, Magnesiumacetat, Aluminiumacetat, Zinkacetat, Bleiacetat, Eisenacetat, Manganacetat, Antimonacetat und Mischungen davon ausgewählt wird.

5. Verfahren nach einem der Ansprüche 1 bis 4, wobei die das Metall enthaltende Verbindung aus: Oxid, Hydroxid, Carbonat, Bicarbonat, Chlorid und Mischungen davon ausgewählt wird.

6. Verfahren nach einem der Ansprüche 1 bis 5, wobei die das Metall enthaltende Verbindung aus: Calciumcarbonat, Calciumbicarbonat, Calciumhydroxid, Magnesiumcarbonat, Magnesiumbicarbonat, Magnesiumhydroxid, Aluminiumhydroxid, Aluminiumtrichlorid.

7. Verfahren nach einem der Ansprüche 1 bis 6, wobei das PET-haltige Material die das Metall enthaltende Verbindung umfasst.

8. Verfahren nach einem der Ansprüche 1 bis 7, wobei das PET-haltige Material Post-Consumer-Abfälle und/oder PET-haltige Post-Industrie-Abfälle, vorzugsweise PET-haltige Post-Consumer-Teppichabfälle, umfasst.

9. Verfahren nach einem der Ansprüche 1 bis 8, wobei das PET-haltige Material in Form von Granulat, Flocken, Fasern oder Flaum vorhanden ist.

10. Verfahren nach einem der Ansprüche 1 bis 9, wobei das Gewichtsverhältnis EG:PETm von 1:1 bis 8:1, vorzugsweise von 1:1 bis 4:1, noch bevorzugter von 1:1 bis 2:1 beträgt, wobei PETm das PET-haltige Material bezeichnet.

## Revendications

1. Procédé de dépolymérisation d'une matière comprenant du polyéthylène téréphtalate (PET) par glycolyse avec de l'éthylène glycol (EG), qui comprend la réaction de la matière comprenant du PET avec de l'EG en présence d'au moins un catalyseur comprenant un acétate de métal afin d'obtenir un produit glycolysé contenant du téréphtalate de bis(2-hydroxyéthyle) (BHET) et/ou des oligomères de celui-ci, le procédé comprenant une étape consistant à préparer ledit catalyseur par réaction in situ entre l'acide acétique et un composé contenant ledit métal.

2. Procédé selon la revendication 1, ladite étape consistant à préparer ledit catalyseur par réaction in situ entre l'acide acétique et le composé contenant ledit métal comprenant :
a. le mélange de l'acide acétique, du composé contenant ledit métal et de l'EG à une température comprise entre 20 - 70°C ;
b. le chauffage du mélange obtenu à l'étape a jusqu'à une température comprise entre 170 et 230°C ;
c. l'ajout de la matière comprenant du PET au mélange obtenu à l'étape b pour obtenir le produit glycolysé.

3. Procédé selon la revendication 2, ladite étape consistant à préparer ledit catalyseur par réaction in situ entre l'acide acétique et le composé contenant ledit métal comprenant :
a. le mélange de l'EG, de l'acide acétique et de la matière comprenant le PET et du composé contenant ledit métal à une température comprise entre 20 et 70°C ;
b. le chauffage du mélange obtenu à l'étape a jusqu'à une température comprise entre 170 et 230°C et faire en sorte que la réaction se produise pour obtenir le produit glycolysé.

4. Procédé selon l'une quelconque des revendications 1 à 3, ledit acétate de métal étant choisi parmi : l'acétate de calcium, l'acétate de magnésium, l'acétate d'aluminium, l'acétate de zinc, l'acétate de plomb, l'acétate de fer, l'acétate de manganèse, l'acétate d'antimoine et leurs mélanges.

5. Procédé selon l'une quelconque des revendications 1 à 4, le composé contenant ledit métal étant choisi parmi : l'oxyde, l'hydroxyde, le carbonate, le bicarbonate, le chlorure et leurs mélanges.

6. Procédé selon l'une quelconque des revendications 1 à 5, dans lequel le composé contenant ledit métal étant choisi parmi : le carbonate de calcium, le bicarbonate de calcium, l'hydroxyde de calcium, le carbonate de magnésium, le bicarbonate de magnésium, l'hydroxyde de magnésium, l'hydroxyde d'aluminium, le trichlorure d'aluminium.

7. Procédé selon l'une quelconque des revendications 1 à 6, le matériau comprenant le PET comprenant le composé contenant ledit métal.

8. Procédé selon l'une quelconque des revendications 1 à 7, la matière comprenant du PET comprenant des déchets de post-consommation et/ou des déchets post-industriels contenant du PET, de préférence des déchets de moquette de post-consommation contenant du PET.

9. Procédé selon l'une quelconque des revendications 1 à 8, la matière comprenant du PET se présentant sous forme de granulés, de flocons, de fibres ou de peluches.

10. Procédé selon l'une quelconque des revendications 1 à 9, le rapport pondéral EG:PETm étant compris entre 1:1 et 8:1, de préférence entre 1:1 et 4:1, plus préférablement encore entre 1:1 et 2:1, PETm désignant la matière comprenant du PET.
